(19) Europäisches Patentamt

European Patent Office

Office européen des brevets

(11) Veröffentlichungsnummer: **0 408 068 A2**

(12) **EUROPÄISCHE PATENTANMELDUNG**

(21) Anmeldenummer: **90113474.2**

(22) Anmeldetag: **13.07.90**

(51) Int. Cl.5: **A61K 31/40**

(30) Priorität: **14.07.89 DE 3923402**

(43) Veröffentlichungstag der Anmeldung:
**16.01.91 Patentblatt 91/03**

(84) Benannte Vertragsstaaten:
**AT BE CH DE DK ES FR GB GR IT LI LU NL SE**

(71) Anmelder: **THERA - Patent Verwaltungs-GmbH**
**Griesberg 2**
**D-8031 Seefeld 1(DE)**

(72) Erfinder: **Die Erfinder haben auf ihre**
**Nennung verzichtet**

(74) Vertreter: **Behrens, Dieter, Dr.-Ing. et al**
**Wuesthoff & Wuesthoff Patent- und**
**Rechtsanwälte Schweigerstrasse 2e 2**
**D-8000 München 90(DE)**

(54) **Verwendung von ACE-Inhibitoren für die Prophylaxe und Therapie der chronisch-venösen Insuffizienz.**

㉗ Die Erfindung betrifft die Verwendung der Inhibitoren des Angiotensin-Umwandlungsenzyms zur Herstellung eines Arzneimittels für die Prophylaxe und Therapie der chronisch-venösen Insuffizienz. Dazu werden die Inhibitoren, beispielsweise in der Form von Captopril, in einer Menge von 0,5 bis 15 mg pro Tag mit üblichen galenischen Hilfs- und Trägerstoffen verabreicht.

EP 0 408 068 A2

## VERWENDUNG VON ACE-INHIBITOREN FÜR DIE PROPHYLAXE UND THERAPIE DER CHRONISCH-VENÖSEN INSUFFIZIENZ

Die Erfindung betrifft die Verwendung der Inhibitoren des Angiotensin-Umwandlungsenzyms zur Herstellung eines Arzneimittels für die Prophylaxe und Therapie der chronisch-venösen Insuffizienz.

60 % der europäischen Bevölkerung leidet an dieser Erkrankung. Insgesamt werden allein in der BRD 1,2 Milliarden an Heilmitteln dafür jährlich ausgegeben. Die ACE-Inhibitoren wie Captopril, Enalapril, Ramipril, Lisinopril u.a. wurden bisher als Mittel zur Behandlung des erhöhten Blutdrucks und der Herzinsuffizienz verwendet sowie in Verbindung mit Extraktstoffen aus enteiweißtem Kälberblut zur Behandlung von Durchblutungs- und Stoffwechselstörungen.

Es ist bekannt, daß Inhibitoren des Angiotensin-Umwandlungsenzyms (Angiotensin-Converting-Enzyme oder ACE) eine starke blutdrucksenkende Wirkung beim essentiellen Hochdruck des Menschen zeigen. Derartige Inhibitoren wurden erstmals aus dem Gift der Schlange Bothrops jararaca isoliert und als Oligopeptide beschrieben; eine entsprechende Wirksamkeit wurde sowohl für das daraus erhaltene Pentapeptid mit der Bezeichnung $B.P.P._{5a}$ als auch für das Nonapeptid der Bezeichnung $B.P.P._{9a}$ oder SQ 20,881, letzteres mit der Aminosäurenfolge pyroGlu-Trp-Pro-Arg-Pro-Gln-Ile-Pro-Pro, nachgewiesen (siehe The Lancet 1973, 1972 und US-A-3 947 575). Ein solcher ACE-Inhibitor ist auch das Prolin-Derivat mit der chemischen Bezeichnung 1- (2S)-3-Mercapto-2-methylpropionyl -L-prolin, das unter der INN-Bezeichnung Captopril im Handel erhältlich ist.

Weitere ACE-Inhibitoren sind unter den Bezeichnungen Enalapril (N-(1-Ethoxycarbonyl-3-phenylpropyl)-L-alanyl-L-prolin), Lisinopril, Zofinopril und Ramipril erhältlich bzw. im arzneimittelrechtlichen Zulassungsverfahren (Zofinopril).

Die Wirkung der ACE-Inhibitoren kann zum einen mit einer Blockierung des Enzyms erklärt werden, welches im Organismus das Angiotensin I in das vasokonstriktorische und blutdruckwirksame Angiotensin II umwandelt. Im weiteren führt die Blockierung des Enzyms zu einer verminderten sympathischen Neurotransmission und letztlich einer Abnahme des Sympathicotonus. Schließlich ist das Conversions-Enzym mit dem kininabbauenden Enzym Kininase II identisch, so daß unter Bedingungen der ACE-Inhibitoren mit einem verminderten Abbau und damit einer gesteigerten Konzentration von vasodilatorisch wirksamen Kininen zu rechnen ist, siehe auch New England Journal of Medicine, 1974, 817 sowie US-A-3 947 575.

Ferner ist bekannt, daß der Zusatz des ACE-Inhibitorpeptids $B.P.P._{9a}$ bzw. SQ 20881 zu bestimmten Extraktstoffen aus dem Bluteiweiß junger Kälber zu einer Verbesserung der mit diesen Extraktstoffen erzielten Wundheilung führt. Dies bedeutet, daß die Wirkung der Extraktstoffe durch den Zusatz der ACE-Inhibitoren verstärkt wird. Die Wirkung kann darauf zurückgeführt werden, daß durch die Kombination der Extraktstoffe mit den ACE-Inhibitoren der Stoffwechsel mangelhaft versorgter Gewebspartien durch eine verstärkte Glukoseaufnahme verbessert wird, wodurch speziell bei diabetischer Stoffwechsellage die Durchblutung verstärkt und, bei lokaler Anwendung, die Wundheilung beschleunigt wird, siehe DE-A-27 29 096 und DE-C-27 59 793.

Es wurde nun überraschend gefunden, daß die ACE-Inhibitoren bei oraler Verabreichung in Mengen, die den Blutdruck nicht mehr wesentlich beeinflussen, die chronisch venöse Insuffizienz im Stadium I und II nach Kappert bei solchen Patienten signifikant verbessern.

Es handelt sich dabei um Patienten mit einer primären Varikose, bei denen infolge einer Klappeninsuffizienz im oberflächen Venensystem die herzwärts gerichtete Blutströmung nicht mehr störungsfrei abläuft. Andererseits kann im Stadium I und II unter Beinarbeit noch eine Druckentlastung erfolgen, da der Zufluß zu den tiefen Venen noch nicht oder erst gering gestört ist. In dieser Situation gibt es bislang außer einer konsequenten Kompressionsbehandlung keine medikamentöse Therapie, die im Stande wäre, das weitere Fortschreiten in Richtung Stadium III und IV bzw. hin zum postthrombotischen Syndrom aufzuhalten. Es ist nun überraschend, daß eine Hemmung des Conversions-Enzyms in niedriger Dosierung ( 2 x 3 mg Captopril/Tag) im Stande ist, die chronisch venöse Insuffizienz im Stadium I und II im Sinne einer Verbesserung des peripheren Venendruckverhaltens unter Belastungsbedingungen günstig zu beeinflussen (Tab. 1).

Die venentonisierende Wirkung war auch überraschend, da die ACE-Hemmer bisher im wesentlichen als vasodilatorische Medikamente angesehen wurden, was ihre Anwendung in der Hochdrucktherapie und in der Herzinsuffizienztherapie erklärt.

Für die erfindungsgemäße Verwendung werden die ACE-Inhibitoren wie Captopril in Mengen von 0,5 bis 15 mg pro Tag vorzugsweise 1 bis 10 mg pro Tag oral verabreicht. Eine typische Dosierung für einen erwachsenen Patienten ist beispielsweise 2 x 3 mg Captopril pro Tag, jedoch kann die Dosierung in Abhängigkeit vom Körpergewicht, Alter und physischen Zustand des Patienten in üblicher Weise variiert werden.

Der Wirkstoff ist für die orale Anwendung üblicherweise mit gängigen Hilfs- und Trägerstoffen formuliert. Dabei liegt die Einzeldosis vorzugsweise im Bereich von 0,5 bis 10 mg, insbesondere 1 bis 5 mg z.B. Captopril.

Bei der Behandlung von Bluthochdruck beträgt die Tagesdosis an Captopril beispielsweise 25 bis 150 mg pro Tag. Sie liegt also weit über der für die Prophylaxe der chronisch-venösen Insuffizienz benötigten täglichen Menge.

Versuchsbericht

Es wurden 25 Patientinnen mit sichtbaren Veränderungen der Beinvenen mit Hilfe der intravasalen peripheren Venendruckmessung unter Ruhe- und Belastungsbedingungen untersucht. Es handelte sich um eine Varikose Grad I und II. Als Belastungsart diente ein während 15 Sek. 10 mal durchgeführter Zehenstand. An Parametern wurde der Ruhedruck, der absolute Druckabfall, die Druckabfallszeit ($t_1$) d.h. die kürzeste Zeit bis zum Erreichen des tiefsten Druckes unter Belastungsbedingungen, sowie die Druckausgleichszeit ($t_2$) d.h. die Zeit, in der nach der Belastung der Ausgangsdruck wieder erreicht wird, gemessen.

Die Patientinnen erhielten nach einer eingangs durchgeführten Phlebodynamometrie 10 Tage lang 2 x 3 mg Captopril täglich, dann wurde die Untersuchung wiederholt. Die Ergebnisse der Versuche sind in der Tabelle I aufgezeichnet. Augehend von einem Ruhedruck, der sich nicht von venengesunden Vergleichskollektiven unterscheidet, kam es während der Belastung mit 10 Zehenständen innerhalb 15 Sek. zu einem absoluten Druckabfall um 45 ± 8 mmHg, entsprechend einem relativen Druckabfall um 51 %. Nach 10 Tagen Therapie mit ACE-Inhibitoren blieb der Ruhedruck unverändert, dagegen fand sich ein absoluter Druckabfall um 52 ± 9 mmHg entsprechend einem relativen Druckabfall um 59 %. (p 0,05 Paarvergleich; Student's t-Test).

Sowohl die Druckabfallszeit als auch die Druckausgleichszeit wiesen in der Tendenz eine Zunahme auf, was für eine Verbesserung der Venentonisierung spricht, jedoch wurde keine statisti sche Signifikanz erreicht. Insgesamt ergibt sich somit nach einer 10-tägigen Behandlung mit einem niedrig dosierten ACE-Hemmer eine deutlich verbesserte Muskelfaszien-Pumpe und damit ein besserer venöser Rückstrom.

Tabelle I

| PERIPHERE PHLEBODYNAMOMETRIE* VOR UND NACH EINER 10-TÄGIGEN BEHANDLUNG MIT ACEINHIBITOREN (ACEI) BEI 25 PATIENTEN MIT CHRONISCH-VENÖSEN INSUFFIZIENZEN IM STADIUM I UND II | | |
|---|---|---|
| | BASAL | NACH 10 TAGEN ACEI |
| Ruhedruck (mmHg) | 88,4 ± 5 | 88,2 ±6 |
| Absoluter Druckabfall (mmHg) | 45 ± 8 | 52 ± 9** |
| Relativer Druckabfall % | 51,0 % | 59,0 % ** |
| Druckabfallszeit $t_1$ (sec) | 8 ± 3 | 8,5 ± 4 |
| Druckausgleichszeit (sec) | 13 ± 5 | 15 ± 5 |

*Belastung mit 10 Zehenständen innerhalb 15 Sekunden.
**p< 0,05; Paarvergleich.

**Ansprüche**

1. Verwendung der Inhibitoren des Angiotensin-Umwandlungsenzyms zur Herstellung eines Arzneimittels für die Prophylaxe und Therapie der chronisch-venösen Insuffizienz.

2. Verwendung nach Anspruch 1, gekennzeichnet durch eine Menge von 0,5 bis 10 mg Wirkstoff je

Dosierungseinheit.